# EUROPEAN PATENT APPLICATION

(11) **EP 3 415 169 A1**
(43) Date of publication of application: **19.12.2018**
(21) Application number: 16889757.7
(22) Date of filing: 05.12.2016
(51) Int. Cl.: A61L 9/01

(54) **DEODORANT**

(30) Priority: 10.02.2016 JP 2016023967
(71) Applicant: Panasonic Intellectual Property Management Co., Ltd., Osaka-shi, Osaka 540-6207 (JP)
(72) Inventor: KAWAGUCHI, Ryousuke, Osaka 540-6207 (JP); SHIBA, Yasuo, Osaka 540-6207 (JP); NAGAYASU, Takahiro, Osaka 540-6207 (JP)
(74) Representative: Müller-Boré & Partner Patentanwälte PartG mbB
(86) International application number: PCT/JP2016/005057
(87) International publication number: WO 2017/138053

(57) **Abstract**

A deodorizer contains: (A) zinc oxide; (B) an organic acid; (C) an aqueous solvent; and (D) an amphoteric surfactant having one of a carboxylate group or a carboxyl group.

## Description

### Technical Field

The present invention relates to deodorizers.

### Background Art

Conventionally, deodorizers are used to reduce various odors generated in daily life such as, for example, unpleasant odors caused by nitrogen compounds, sulfur compounds, short chain fatty acids, and aldehydes. Also, in recent years, improvements in housing conditions include higher airtightness, which make people more sensitive toward unpleasant odors caused by chemical compounds, for example, originated in building materials. Accordingly, deodorizers which can remove unpleasant odors more effectively are desired.

In addition, since deodorizers might come in contact with human bodies when in use and are discarded after use, low-toxic deodorizers are desired.

JP 2001-190344A (hereinafter also referred to as "related art document") proposes a deodorizer containing zinc oxide and an amino acid as deodorizing components and water as a solvent. In this deodorizer, zinc oxide which is hardly soluble in water is dissolved in water, being facilitated by the amino acid as a solubilizer.

### Summary of Invention

### Technical Problem

However, in the deodorizer disclosed in the related art document, solubility of zinc oxide in water is not sufficient, leading to possibilities of poor transparency of the solution and floating particles and precipitation of zinc oxide in the solution. Due to this, it is concerned that product value is decreased due to poor appearance. Also there exist restrictions on how-to-use.

In the above explained circumstances, the present invention aims to provide a low-toxic deodorizer which has high solution transparency and prevented occurrence of floating particles and precipitation.

### Solution to Problem

In order to solve the above technical problem, a deodorizer according to the present invention contains: (A) zinc oxide; (B) an organic acid; (C) an aqueous solvent: and (D) an amphoteric surfactant having one of a carboxylate group or a carboxyl group.

### Advantageous Effects of Invention

A deodorizer according to the present invention is low-toxic, and has high solution transparency and prevented occurrence of floating particles and precipitation.

### Description of Embodiments

Hereinafter, one embodiment of the present invention is explained.

A deodorizer of the present embodiment contains: (A) zinc oxide; (B) an organic acid; (C) an aqueous solvent; and (D) an amphoteric surfactant having one of a carboxylate group or a carboxyl group. (D) The amphoteric surfactant has one of a carboxylate group or a carboxyl group, but does not make (A) the zinc oxide soluble in (C) the aqueous solvent such as water without existence of (B) the organic acid such as amino acid. However, by using (D) the amphoteric surfactant in combination with (B) the organic acid which functions as a solubilizer for (A) the zinc oxide, (D) the amphoteric surfactant functions as a solubilizing facilitator for increasing solubility of (A) the zinc oxide in (C) the aqueous solvent. Therefore, using (D) the amphoteric surfactant can increase solubility of (A) the zinc oxide, leading to improved solution transparency and prevented occurrence of floating particles and precipitation.

Further, since (D) the amphoteric surfactant is low-toxic, high safety of the deodorizer which might come in contact with human bodies when in use and is discarded after use is realized.

A kind of (D) the amphoteric surfactant is not especially limited, but examples of (D) the amphoteric surfactant include a betaine-type amphoteric surfactant and a glycine-type amphoteric surfactant. One kind of the amphoteric surfactant may be used alone, and two or more kinds of the amphoteric surfactants may be used in combination.

Examples of the betaine-type amphoteric surfactant include an alkyl betaine. Examples of the alkyl betaine include lauryl dimethyl amino acetic acid betaine and stearyl dimethyl amino acetic acid betaine. One kind of the betaine-type amphoteric surfactant may be used alone, and two or more kinds of the betaine-type amphoteric surfactants may be used in combination.

Examples of the glycine-type amphoteric surfactant include an amphoteric surfactant represented by the following general formula (1) and an amphoteric surfactant represented by the following general formula (2). One kind of the glycine-type amphoteric surfactant may be used alone, and two or more kinds of the glycine-type amphoteric surfactants may be used in combination.

R-(NHCH₂CH₂)₂NHCH₂COOM (1)

(R-NHCH₂CH₂)₂NCH₂COOM (2)

(In the above formula, R represents an alkyl group or an alkenyl group of 3 to 20 carbons and M represents a hydrogen atom, an alkali metal, amine, or alkanol amine.)

Examples of the glycine-type amphoteric surfactants represented by the above general formula (1) and (2) include sodium lauryl diamino ethyl glycine (product name: LEBON S, manufactured by Sanyo Chemical Industries, Ltd.) and hydrochloric acid alkyl polyamino ethyl glycine (product name: LEBON U, manufactured by Sanyo Chemical Industries, Ltd.).

(D) The amphoteric surfactant preferably has same numbers of monovalent cation and monovalent anion within molecule. Using (D) the amphoteric surfactant having same numbers of monovalent cation and monovalent anion within molecule can increase solution transparency and prevent occurrence of floating particles and precipitation more. Examples of (D) the amphoteric surfactant having same numbers of monovalent cation and monovalent anion within molecule include a betaine-type amphoteric surfactant.

Lauryl dimethyl amino acetic acid betaine which can improve solution transparency more and prevent occurrence of floating particles and precipitation furthermore is preferably used as (D) the amphoteric surfactant.

In order to improve solution transparency and prevent occurrence of floating particles and precipitation, an amount of (D) the amphoteric surfactant having a carboxylate group or a carboxyl group is preferably within a range of 0.2 to 2 weight % in the deodorizer of the present embodiment.

A kind of (A) the zinc oxide in the deodorizer of the present embodiment is not especially limited. Examples of (A) the zinc oxide include powdery zinc oxide which is used as cosmetic and medical materials such as fine zinc oxide.

(B) The organic acid in the deodorizer of the present embodiment functions as a deodorizing component and a solubilizer for (A) the zinc oxide. Examples of (B) the organic acid include amino acid and pyrrolidone carboxylic acid. One kind of the organic acid may be used alone, and two or more kinds of the organic acids may be used in combination. In terms of deodorizing function, solubility of (A) the zinc oxide, and solution safety, amino acid is preferably used as (B) the organic acid. Examples of the amino acid include glycine, alanine, phenyl alanine, glutamate, proline, betaine, and sarcosine. One kind of the amino acid may be used alone, and two or more kinds of the amino acids may be used in combination. Also, any isomers of the amino acid such as D-enantiomer and L-enantiomer as well as racemate of the amino acid can be used. Among these amino acids, glycine, alanine, and sarcosine are preferable.

Taking deodorizing function and solubility of (A) the zinc oxide into account, a mass ratio of an amount of (A) the zinc oxide to an amount of (B) the organic acid is preferably within a range of 1 : 3 to 1 : 40 in the deodorizer of the present embodiment.

(C) The aqueous solvent in the deodorizer of the present embodiment functions as a solvent for (A) the zinc oxide, (B) the organic acid, and (D) the amphoteric surfactant. Examples of (C) the aqueous solvent include: water such as deionized water and tap water; and a mixed solvent of water and an aqueous organic solvent such as a water-alcohol solvent, a water-glycol solvent, and a water-glycol monoether solvent. Among these solvents, water is preferable in terms of safety and cost.

Taking deodorizing function and prevention of occurrence of floating particles and precipitation, a total amount of (A) the zinc oxide and (B) the organic acid is preferably within a range of 0.01 to 20 weight%, and more preferably within a range of 0.1 to 10 weight%, with respect to an amount of (C) the aqueous solvent.

It is preferable that the deodorizer of the present embodiment further contains (E) an organic acid metal salt. Adding (E) the organic acid metal salt to the deodorizer can improve solution transparency further. It is considered that this improvement in solution transparency stems from complex formation which leads to more improved stability of (A) the zinc oxide. Note that adding an inorganic metal salt does not contribute to improvement in solution transparency. A kind of (E) the organic acid metal salt is not especially limited. Examples of (E) the organic acid metal salt include sodium dehydroacetate, trisodium citrate, and potassium sorbate. One kind of the organic acid metal salt may be used alone, and two or more kinds of the organic acid metal salts may be used in combination.

Additives may be appropriately added to the deodorizer of the present embodiment as long as the effects of the deodorizer are not inhibited. Examples of the additive include a preservative, a refined oil, a fragrancing agent and an ultraviolet-absorbing agent. One kind of additive may be used alone, and two or more kinds of additives may be used in combination.

The deodorizer of the present embodiment can be prepared, for example, by following method. A suspension of (A) the zinc oxide and an aqueous solution of (B) the organic acid are prepared in advance, which are then mixed at a predetermined ratio to obtain the deodorizer. The deodorizer also can be prepared by mixing fine particles of (A) the zinc oxide and (B) the organic acid at a predetermined ratio and then dissolving the mixture in (C) the aqueous solvent. The deodorizer also can be prepared by adding fine particles of (A) the zinc oxide into an aqueous solution of (B) the organic acid at a predetermined ratio.

The deodorizer of the present embodiment can be used in various forms such as spray, gel with gel base, and aerosol, as well as in a form of aqueous solution, depending on the condition of use.

The deodorizer of the present embodiment can be used to remove gas with unpleasant odor caused by nitrogen compounds such ammonia and amines, sulfur compounds such hydrogen sulfide and methyl mercaptan, short chain fatty acids such as acetate, isovaleric acid, and caproic acid, and aldehydes such as formaldehyde and acetaldehyde.

The deodorizer of the present embodiment explained above contains (D) the amphoteric surfactant having one of a carboxylate group or a carboxyl group in addition to (A) the zinc oxide, (B) the organic acid, and (C) the aqueous solvent. Accordingly the deodorizer is low-toxic and has high solution transparency and prevented occurrence of floating particles and precipitation.

The present invention is not limited to the above explained embodiment.

### Examples

Hereinafter, the present invention is explained further in detail, referring to examples; however, the present invention is not limited to the examples. Note that an amount of each component listed in Tables 1 to 3 is in weight%.

### 1. Evaluation of deodorizers containing various surfactants

Each component listed in Table 1 was added to deionized water which corresponds to (C) the aqueous solvent at an amount presented in Table 1 at room temperature. The mixture was stirred and the deodorizer was obtained without filtration. Each deodorizer contains (A) the zinc oxide, (B) the organic acid, and the preservative. The deodorizer of example 1 further contains lauryl dimethyl amino acetic acid betaine which is an amphoteric surfactant having a carboxylate group, as the surfactant. The deodorizer of example 2 further contains sodium lauryl diamino ethyl glycine which is an amphoteric surfactant having a carboxyl group, as the surfactant. The deodorizer of comparative example 2 further contains sodium polyoxyethylene alkyl ether sulfate which is an anion surfactant, as the surfactant. The deodorizer of comparative example 3 further contains cetyl pyridium chloride which is a cation surfactant, as the surfactant. The deodorizer of comparative example 4 further contains polyoxy alkyl decyl ether which is a nonion surfactant, as the surfactant. The deodorizer of comparative example 1 does not contain any surfactant.

Following evaluation tests were carried out for each of the obtained deodorizers.

### (Solution appearance)

As described above, the components of each deodorizer was added to deionized water and the mixture was stirred. After sitting the obtained solution still for 30 minutes, solution appearance was evaluated as following. In this evaluation test, "excellent" and "good" are considered satisfactory, and "fair" and "bad" are considered unsatisfactory.
excellent: colorless, transparent, and no floating particles nor precipitation
good: pale blue white color, semi-transparent, and no floating particles nor precipitation
fair: some floating particles but no precipitation
bad: white-turbid, and precipitation

### (Toxicity of surfactant)

Each of the surfactants listed in Table 1, a value of oral LD 50 according to a product catalog was presented.

### (Deodorizability)

Each deodorizer solution was diluted with deionized water to obtain a 200-fold dilution which was used to measure deodorizability toward hydrogen sulfide.

Deodorizability was measured by the following method, using high concentrations gaseous hydrogen sulfide (0.15 ml).

A piece of nonwoven cloth was put into a flask and impregnated with the dilution of the deodorizer. Then, hydrogen sulfide was supplied to the flask. After 60 minutes, gas within the flask was absorbed using a gas detection tube and a concentration of hydrogen sulfide in the absorbed gas was measured. Note that deodorizability was determined by comparison to a concentration of hydrogen sulfide in a flask which was supplied with hydrogen sulfide but did not contain the dilution of the deodorizer.

As for results of the measurements of deodorizability toward hydrogen sulfide as explained above, deodorizability of 90 % or more toward hydrogen sulfide was evaluated as "good".

All the results of the measurements and evaluations are represented in Table 1.

**[Table 1]**

| | | Example 1 | Example 2 | Comparative example 1 | Comparative example 2 | Comparative example 3 | Comparative example 4 |
|---|---|---|---|---|---|---|---|
| (A) Zinc oxide*¹ | | 1 | 1 | 1 | 1 | 1 | 1 |
| (B) Organic acid | Glycine*² | 4.5 | 4.5 | 4.5 | 4.5 | 4.5 | 4.5 |
| Surfactant | LEABON LD-36*³ (pureness 41 weight%) Lauryl dimethyl amino acetic acid betaine | 5 | | | | | |
| | LEABON S*⁴ (pureness 30 weight%) Sodium lauryl diamino ethyl glycine | | 5 | | | | |
| | Emal (registered trademark) D-4-D*⁵ (pureness 35 weight%) Sodium polyoxyethylene alkyl ether sulfate | | | | 5 | | |
| | Cetyl pyridium chloride | | | | | 1.8 | |
| | NOIGEN (registered trademark) XL-140*⁶ Polyoxy alkyl decyl ether | | | | | | 1.8 |
| Preservative* ⁷ | | 0.1 | 0.1 | 0.1 | 0.1 | 0.1 | 0.1 |
| (C) Aqueous solvent | Deionized water | 89.4 | 89.4 | 94.4 | 89.4 | 92.6 | 92.6 |
| Total (weight%) | | 100.0 | 100.0 | 100.0 | 100.0 | 100.0 | 100.0 |
| Solution appearance (sat still for 30 minutes after stirring) | | excellent | good | bad | bad | excellent | good |
| | | colorless transparent no floating particles no precipitation | pale blue white semi- transparent no floating particles no precipitation | blue white semi-transparent precipitation | white-turbid | colorless transparent no floating particles no precipitation | pale blue white semi- transparent no floating particles no precipitation |
| Property of surfactant | | Amphoteric | Amphoteric | (None) | Anion (EO) | Cation | Nonion |
| Toxicity of surfactant (oral LD50) | | >2500mg | 2930mg | | 300-2000mg | 200mg | 200-2000mg |
| Deodorizability (200-fold diluent) | Deodorizability (hydrogen sulfide, %) | 100% | 100% | 100% | - | 100% | 100% |
| | Evaluation | good | good | good | - | good | good |

| | | | | | | | |
|---|---|---|---|---|---|---|---|
| *1 Fine particle zinc oxide (manufactured by SAKAI CHEMICAL INDUSTRY CO., LTD.) *2 Glycine M (manufactured by FUSO CHEMICAL CO., LTD.) *3 manufactured by Sanyo Chemical Industries, Ltd. *4 manufactured by Sanyo Chemical Industries, Ltd. *5 manufactured by Kao Corporation *6 manufactured by DKS Co. Ltd *7 manufactured by SAN NOPCO LIMITED | | | | | | | |

As shown in Table 1, good solution appearances were observed in the examples 1 and 2 which contain the amphoteric surfactant, the comparative example 3 which contains the cation surfactant, and the comparative example 4 which contains the nonion surfactant, compared to the comparative example 1 which does not contain the surfactant. The example 1 which contains the amphoteric surfactant of lauryl dimethyl amino acetic acid betaine and the comparative example 3 which contains the cation surfactant of cetyl pyridium chloride exhibited the best results of colorless, transparent, and no floating particles. The deodorizabilities of the examples 1 and 2 and the comparative examples 3 and 4 were as good as the comparative example 1 which does not contain the surfactant. The comparative example 2 which contain the anion surfactant exhibited white-turbid and solution transparency was lower than the comparative example 1 which does not contain the surfactant.

As for the toxicities of the surfactants, the oral LD50 values of the examples 1 and 2 which contain the amphoteric surfactant were higher than 2500 mg, showing low-toxicities. On the other hand, the oral LD50 values of the comparative examples 2, 3, and 4 which respectively contain the anion surfactant, the cation surfactant, and the nonion surfactant were less than 2000 mg, showing high-toxicities.

Considering the above results of solution appearance, toxicity, and deodorizability comprehensively, the evaluation of the example 1 which contains the amphoteric surfactant of lauryl dimethyl amino acetic acid betaine was the best of all. Considering the results of solution appearance and toxicity comprehensively, although the deodorizer of the example 2 which contains the amphoteric surfactant of sodium lauryl diamino ethyl glycine exhibits less excellent solution appearance compared to the example 1, the deodorizer of the example 2 exhibited improved solution appearance compared to the comparative example 1 which does not contain the surfactant without increasing toxicity.

### 2. Evaluation of deodorizers containing various amphoteric surfactants

Since the examples 1 and 2 which contain the amphoteric surfactant exhibited good solution appearance, low-toxicity of the surfactant, and good deodorizability in the above evaluation tests, further evaluations were carried out to investigate a relationship between a kind of amphoteric surfactant and solution appearance.

Each component listed in Table 2 was added to deionized water which corresponds to (C) the aqueous solvent at an amount presented in Table 2 at room temperature. The mixture was stirred and the deodorizer was obtained without filtration. Each deodorizer contains (A) the zinc oxide, (B) the organic acid, and the preservative. The deodorizer of example 1 further contains lauryl dimethyl amino acetic acid betaine which is an amphoteric surfactant having a carboxylate group. The deodorizer of example 2 further contains sodium lauryl diamino ethyl glycine which is an amphoteric surfactant having a carboxyl group. The deodorizer of comparative example 5 further contains lauramidopropyl hydroxy sultaine which is an amphoteric surfactant not having a carboxylate group nor a carboxyl group. The deodorizer of comparative example 1 does not contain any surfactant.

Following evaluation tests were carried out for each of the obtained deodorizers.

### (Solution appearance)

As described above, the components of each deodorizer was added to deionized water and the mixture was stirred. After sitting the obtained solution still for 30 minutes, solution appearance was evaluated as following. In this evaluation test, "excellent" and "good" are considered satisfactory, and "fair" and "bad" are considered unsatisfactory.
excellent: colorless, transparent, and no floating particles nor precipitation
good: pale blue white color, semi-transparent, and no floating particles nor precipitation
fair: some floating particles but no precipitation
bad: white-turbid, and precipitation

The results of the above evaluation tests are shown in Table 2.

**[Table 2]**

| | | Example 1 | Example 2 | Comparative example 1 | Comparative example 5 |
|---|---|---|---|---|---|
| (A) Zinc oxide*¹ | | 1 | 1 | 1 | 1 |
| (B) Organic acid | Glycine*² | 4.5 | 4.5 | 4.5 | 4.5 |
| Surfactant | LEABON LD-36*³ (pureness 41 weight%) Lauryl dimethyl amino acetic acid betaine | 5 | | | |
| | LEABON S*⁴ (pureness 30 weight%) Sodium lauryl diamino ethyl glycine | | 5 | | |
| | SOFTAZOLINE LSB (pureness 29 weight%)*⁵ Lauramidopropyl hydroxy sultaine | | | | 5 |
| Preservative*⁶ | | 0.1 | 0.1 | 0.1 | 0.1 |
| (C) Aqueous solvent | Deionized water | 89.4 | 89.4 | 94.4 | 89.4 |
| Total (weight%) | | 100.0 | 100.0 | 100.0 | 100.0 |
| Hydrophobic group | | C₁₂H₂₅ | C₁₂H₂₅ | | C₁₁H₂₃ |
| Hydrophilic group (anion) | | COO⁻ | COO⁻ | | SO₃⁻ |
| Hydrophilic group (cation) | | N⁺ | (N⁺)×3 | | N⁺ |
| Solution appearance (sat still for 30 minutes after stirring) | | excellent | good | bad | fair |
| | | colorless transparent no floating particles no precipitation | pale blue white semi- transparent no floating particles no precipitation | blue white semi-transparent precipitation | colorless transparent white floating particles |

| | | | | | |
|---|---|---|---|---|---|
| *1 Fine particle zinc oxide (manufactured by SAKAI CHEMICAL INDUSTRY CO., LTD.) *2 Glycine M (manufactured by FUSO CHEMICAL CO., LTD.) *3 manufactured by Sanyo Chemical Industries, Ltd. *4 manufactured by Sanyo Chemical Industries, Ltd. *5 manufactured by Kawaken Fine Chemicals Co., Ltd. *6 manufactured by SAN NOPCO LIMITED | | | | | |

As shown in Table 2, the example 1 which contains the amphoteric surfactant of lauryl dimethyl amino acetic acid betaine having a carboxylate group was colorless, transparent, and floating particle-free, showing the best results. The example 2 which contains the amphoteric surfactant of sodium lauryl diamino ethyl glycine having a carboxyl group exhibited less excellent solution transparency compared to the example 1, but was free from white floating particles and had improved solution appearance compared to the comparative example 1 which does not contain the surfactant. On the other hand, the comparative example 5 which contains the amphoteric surfactant of lauramidopropyl hydroxy sultaine was colorless but had white floating particles and exhibited poor solution appearance.

As shown by the above results, the examples 1 and 2 which contain a weakly acidic amphoteric surfactant, i.e., (D) the amphoteric surfactant having a carboxylate group (-COO⁻) or a carboxyl group exhibited improved solution appearance. Especially, the example 1 which contains the amphoteric surfactant having same numbers of monovalent cation (N⁺) and monovalent anion (COO⁻) within molecule exhibited better solution appearance compared to the example 2 which contains the amphoteric surfactant having different numbers of monovalent cation and monovalent anion within molecule. On the other hand, the comparative example 5 which contains a strongly acidic surfactant having a sulfo group (-SO₃⁻) exhibited white floating particles and unsatisfactory solution appearance.

### 3. Amounts of amphoteric surfactants

Taking the above results into account, further evaluation of solution appearance was carried out by varying the amount of (D) the amphoteric surfactant having a carboxylate group or a carboxyl group which contributes to good solution appearance, low-toxicity of the surfactant, and good deodorizability.

Each component listed in Table 3 was added to deionized water which corresponds to (C) the aqueous solvent at an amount presented in Table 3 at room temperature. The mixture was stirred and the deodorizer was obtained without filtration. After sitting the obtained solution still for 30 minutes, solution appearance was evaluated. The solution which is colorless and transparent and exhibits no precipitation was evaluated as "excellent".

The results of the above evaluation tests are shown in Table 3

**[Table 3]**

| | | Example 3 | Example 4 | Example 5 | Example 6 |
|---|---|---|---|---|---|
| (A) Zinc oxide*¹ | | 0.8 | 0.8 | 0.8 | 0.8 |
| (B) Organic acid | Glycine*² | 3.6 | 3.6 | 3.6 | 3.6 |
| (D) Amphoteric Surfactant | LEABON LD-36*³ (pureness 41 weight%) Lauryl dimethyl amino acetic acid betaine | 5 | 2.5 | 1 | 0.5 |
| Preservative *⁴ | | 0.1 | 0.1 | 0.1 | 0.1 |
| (C) Aqueous solvent | Deionized water | 90.5 | 93 | 94.5 | 95 |
| Total (weight%) | | 100.0 | 100.0 | 100.0 | 100.0 |
| Amount of surfactant (weight% by pure content) | | 2.1% | 1.0% | 0.4% | 0.2% |
| Solution appearance (sat still for 30 minutes after stirring) | | excellent | excellent | excellent | excellent |
| | | colorless transparent no floating particles no precipitation | colorless transparent no floating particles no precipitation | colorless transparent no floating particles no precipitation | colorless transparent no floating particles no precipitation |

| | | | | | |
|---|---|---|---|---|---|
| *1 Fine particle zinc oxide (manufactured by SAKAI CHEMICAL INDUSTRY CO., LTD.) *2 Glycine M (manufactured by FUSO CHEMICAL CO., LTD.) *3 manufactured by Sanyo Chemical Industries, Ltd. *4 manufactured by SAN NOPCO LIMITED | | | | | |

As shown in Table 3, examples 3, 4, 5, and 6 which contain lauryl dimethyl amino acetic acid betaine as (D) the amphoteric surfactant having a carboxylate group or a carboxyl group at amounts within a range of 0.2 to 2 weight% were colorless and transparent with no precipitation and exhibited good solution appearance.

## Claims

1. A deodorizer comprising:
(A) zinc oxide;
(B) an organic acid;
(C) an aqueous solvent; and
(D) an amphoteric surfactant having one of a carboxylate group or a carboxyl group.

2. The deodorizer according to claim 1, wherein
(D) the amphoteric surfactant has same numbers of monovalent cation and monovalent anion within molecule.

3. The deodorizer according to claim 1 or 2, wherein
(D) the amphoteric surfactant is lauryl dimethyl amino acetic acid betaine.
